# EUROPÄISCHE PATENTSCHRIFT

(11) **EP 0 648 274 B1**
(45) Veröffentlichungstag und Bekanntmachung des Hinweises auf die Patenterteilung: **28.08.1996**
(21) Anmeldenummer: 93912954.0
(22) Anmeldetag: 11.06.1993
(51) Int. Cl.: C12P 7/54

(54) **VERFAHREN ZUR HERSTELLUNG VON 3-HYDROXYPHENYLESSIGSÄURE**
METHOD OF PREPARING 3-HYDROXYPHENYLACETIC ACID
PROCEDE DE PRODUCTION D'ACIDE 3-HYDROXYPHENYLACETIQUE

(30) Priorität: 20.06.1992 DE 4220241
(43) Veröffentlichungstag der Anmeldung: 19.04.1995
(73) Patentinhaber: BASF Aktiengesellschaft, 67063 Ludwigshafen (DE)
(72) Erfinder: STAUDENMAIER, Horst Ralf, D-67134 Birkenheide (DE); HAUER, Bernhard, D-6701 Fussgoenheim (DE); LADNER, Wolfgang, D-6701 Fussgoenheim (DE); MUELLER, Ursula, D-6701 Fussgoenheim (DE); PRESSLER, Uwe, D-6701 Altrip (DE); MEYER, Joachim, D-6701 Maxdorf (DE)
(86) Internationale Anmeldenummer: EP9301484
(87) Internationale Veröffentlichungsnummer: WO9400553

(56) Entgegenhaltungen:
- PHYTOPATHOLOGY, Band 60, Juni 1970, St. Paul, MN (US); K. KOHMOTO et al., Seiten 1025-1027
- JOURNAL OF BACTERIOLOGY, Band 141, Nr. 2, Februar 1980, Washington, DC (US); J.J. ANDERSON et al., Seiten 534-543

## Beschreibung

Die Erfindung betrifft ein neues Verfahren zur Herstellung von 3-Hydroxyphenylessigsäure.

Es ist bekannt, daß eine Reihe von Mikroorganismen in der Lage ist, Phenylessigsäure zu hydroxylieren.

Dabei entsteht neben verschiedenen anderen Hydroxylierungsprodukten auch 3-Hydroxyphenylessigsäure, die jedoch als Zwischenprodukt im Stoffwechsel der Mikroorganismen weiter abgebaut wird. (J. J. Anderson et al., J. Bacteriol, 141, 534-543 (1980)).

Von Keisuke Kohmoto et al. (Phytopathology, 60, 1025-1026 (1970)) wurde beschrieben, daß der Pilz Rhizoctonia solani Phenylessigsäure in meta-Position hydroxylieren kann. Zur Herstellung von 3-Hydroxyphenylessigsäure wurde zuerst der Pilz gezüchtet, anschließend die Biomasse isoliert und mit einer Lösung von Phenylessigsäure inkubiert ("Replacement-Kultur"). Dabei wurden als maximale Konzentration 2,5 g/l 3-Hydroxyphenylessigsäure erhalten.

Diese Herstellung eignet sich aufgrund der geringen Produktkonzentration und der aufwendigen "Replacement-Kultur" nicht für ein technisches Verfahren.

Es bestand daher die Aufgabe, ein fermentatives Herstellungsverfahren für 3-Hydroxyphenylessigsäure aus Phenylverbindungen bereitzustellen, das einfach durchzuführen ist und gute Ausbeuten liefert.

Demgemäß wurde gefunden, daß das eingangs genannte Verfahren zur Herstellung von 3-Hydroxyphenylessigsäure besonders vorteilhaft verläuft, wenn man einen Pilz der Gattung Rhizoctonia, Ceratobasidium oder Pellicularia in Gegenwart eines üblichen Nährmediums, dem eine Phenylverbindung der allgemeinen Formel I, 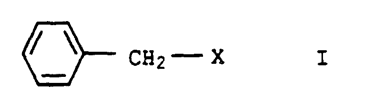 in der der Substituent folgende Bedeutung hat:
X = COOR, CH₂OH, CH₂NH₂, 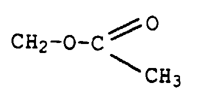
R = Methyl, Ethyl, Wasserstoff, Natrium, Kalium, Ammonium,
zugesetzt wird, züchtet.

Pilze der Gattungen Rhizoctonia, Ceratobasidium und Pellicularia sind bekannt (D. L. Hawksworth, B. C. Sutton and G. C. Ainsworth: Ainsworth & Bisby's Dictionary of the Fungi, Seventh Edition, 1983 und hierin zitierte Literatur).

Die für das erfindungsgemäße Verfahren besonders geeignete Art ist Rhizoctonia solani.

Geeignete Nährmedien zur Züchtung der Pilze sind solche, die Kohlenstoffquellen, Stickstoffquellen, anorganische Salze und gegebenenfalls geringe Mengen an Spurenelementen und Vitaminen enthalten. Als Stickstoffquellen können anorganische bzw. organische Stickstoffverbindungen oder Materialien, die diese Verbindungen enthalten, verwendet werden. Beispiele sind Ammoniumsalze, Nitrate, Maisquellwasser, Bierhefeautolysat, Sojabohnenmehl, Weizengluten, Hefeextrakt, Hefe, Harnstoff und Kartoffelprotein. Als Kohlenstoffquellen können beispielsweise Zucker wie Glucose, Polyole wie Glycerin oder Fette wie Sojaöl verwendet werden.

Beispiele für anorganische Salze sind die Salze von Calcium, Magnesium, Mangan, Kalium, Zink, Kupfer, Eisen und anderen Metallen. Als Anion der Salze ist besonders das Phosphation zu nennen. Gegebenenfalls werden dem Nährmedium Wachstumsfaktoren zugesetzt, wie z.B. Biotin, Riboflavin oder andere Vitamine.

Der gute Erfolg des Verfahrens hängt nicht erkennbar von der Art des Nährmediums ab.

Dem Nährmedium wird für das erfindungsgemäße Verfahren eine Phenylverbindung zugesetzt. Als Phenylverbindungen können in diesem Verfahren Phenylessigsäureverbindungen wie die freie Phenylessigsäure, ihre Salze, insbesondere die entsprechenden Alkalisalze, wobei das Natrium- und das Kaliumsalz besonders bevorzugt ist, oder Ester der Phenylessigsäure, bevorzugt der Methyl- oder Ethylester, eingesetzt werden. Bei der Verwendung von Estern als Edukt wird durch das erfindungsgemäße Verfahren nicht nur eine Hydroxylierung in meta-Position, sondern auch eine Spaltung des Esters bewirkt, so daß als Produkt 3-Hydroxyphenylessigsäure entsteht.

Weitere geeignete Phenylverbindungen sind 2-Phenylethanol, 2-Phenylethylamin und Essigsäure-2-phenylethylester.

Bei diesen Substraten wird durch das erfindungsgemäße Verfahren zusätzlich zur Hydroxylierung in meta-Position auch eine Carboxylgruppe gebildet, so daß als Produkt 3-Hydroxyphenylessigsäure entsteht.

Die besonders bevorzugten Phenylverbindungen sind das Natrium- und das Ammoniumsalz der Phenylessigsäure.

Die Phenylverbindungen werden in der Regel in solchen Mengen zugegeben, daß eine Konzentration von etwa 1 bis 50 g, bevorzugt von 5 bis 30 g, pro Liter Nährmedium eingestellt wird.

Die Phenylverbindungen können dem Nährmedium am Anfang der Züchtung des Pilzes oder während der Züchtung in mehreren Portionen oder kontinuierlich zugegeben werden.

Außerdem ist es möglich, den gezüchteten Pilz nach beendetem Umsatz zurückzubehalten und ihn in frischem Nährmedium mit der Phenylverbindung weiterzuzüchten.

Diese Form der Wiederverwendung der Biomasse ist eine besonders vorteilhafte Ausgestaltung des erfindungsgemäßen Verfahrens, da dabei beispielsweise die Anzuchtszeit für die Biomasse eingespart wird.

Die Züchtung des Pilzes bedarf keiner weiteren besonderen Bedingungen. So kann die Züchtungstemperatur in der Regel zwischen 20 und 40°C liegen, bevorzugt wird eine Temperatur zwischen 25 und 35°C gewählt.

Der pH-Wert des Fermentationsmediums wird zwischen 3 und 9 festgehalten. Vorteilhaft sind pH-Werte zwischen 4 und 7.

Die Fermentationszeiten betragen üblicherweise zwischen 1 und 10 Tagen, um eine maximale Anreicherung des Produkts im Fermentationsmedium zu erreichen.

Der Reaktionsumsatz kann leicht durch Probenentnahme und beispielsweise gaschromatographische Analyse festgestellt werden.

Die Erfindung wird durch die folgenden Beispiele weiter veranschaulicht.

### Beispiel 1

Überprüfung verschiedener Pilzstämme auf Umsetzung von Phenylessigsäure zu 3-Hydroxyphenylessigsäure

### Medium 1

| | |
|---|---|
| 10 g/l | Glucose |
| 40 g/l | Maisquellwasser |
| 1,5 g/l | KH₂PO₄ |
| 3,6 g/l | K₂HPO₄ |
| pH 6,8 | |

Die Phenylessigsäure wurde unter Zugabe von wenig verdünnter NaOH in Wasser gelöst und sterilfiltriert.

Je 30 ml Medium 1 mit 1 g/l Phenylessigsäure wurden in sterile 250 ml-Erlenmeyerkolben mit einer Schikane gefüllt. Die Kolben wurden mit je einem 0,5 x 0,5 cm großen Stück Agar beimpft, das aus einer mit dem jeweiligen Stamm bewachsenen Agarplatte ausgestochen wurde. Die Kolben wurden bei 25°C und 180 U/min geschüttelt. Nach 3 bzw. 7 Tagen wurde 1 ml des Kulturüberstandes abgenommen, mit 100 µl 5 N Salzsäure und 800 µl Essigsäureethylester versetzt und 15 s kräftig gemischt. 700 µl der Essigsäureethylesterphase wurden abgenommen und bei 50°C unter einem leichten Stickstoffstrom eingedampft. Der Rückstand wurde in 70 µl Essigsäureethylester gelöst und 50 µl davon in ein Probegläschen für die Gaschromatographie überführt. Dazu wurden 50 µl N-Methyl-N-trimethylsilyltrifluoracetamid (MSTFA) gegeben und gemischt. Die Proben wurden gaschromatographisch untersucht. Als Vergleich diente eine authentische Probe 3-Hydroxyphenylessigsäure.

Die Ergebnisse sind in der Tabelle zusammengefaßt.

**Tabelle:**

| Umsatz von Phenylessigsäure zu 3-Hydroxyphenylessigsäure | | |
|---|---|---|
| Stamm | Umsatz in % nach | |
| | 3 Tagen | 7 Tagen |
| Rhizoctonia solani Lu 6467 | 50,5 | 11,9 * |
| Rh. solani LU 6464 | 0,0 | 0,8 |
| Rh. solani LU 6474 | 12,5 | 6,4 * |
| Rh. solani LU 6473 | 22,3 | 55,7 |
| Rh. solani LU 6475 | 14,7 | 80,2 |
| Rh. solani LU 6476 | 48,1 | 28,0 * |
| Rh. solani DSM 852 (= ATCC 13250) | 95,5 | 94,3 |
| Rh. solani DSM 63010 (= IBM 11662) | 1,5 | 4,7 |
| Rh. solani DSM 843 (= ATCC 13249) | 51,6 | 63,1 |
| Rh. solani ATCC 10159 | 3,0 | 35,0 |
| Rh. solani ATCC 16116 | 2,0 | 94,0 |
| Rh. solani ATCC 48804 (= CMI 34886) | 57,0 | 79,0 |
| Rh. solani ATCC 62153 | 32,0 | 86,0 |
| Rh. solani ATCC 62154 | 0,0 | 25,0 |
| Rh. solani ATCC 62156 | 80,0 | 97,0 |
| Rh. tuliparum LU 6470 | 0,0 | 1,6 |
| Rh. cerealis LU 6471 | 1,8 | 33,5 |
| Rh. muneratii DSM 903 (= ATCC 13247) | 7,0 | 34,0 |
| Pellicularia filamentosa ATCC 13289 | 2,0 | 61,0 |
| Pellicularia filamentosa ATCC 13290 | 2,0 | 60,0 |
| Ceratobasidium cornigerum CBS 137.82 (= ATCC 13247) | 13,7 | 13,3 |

| | | |
|---|---|---|
| * Abbau des Produkts | | |

Die mit LU bezeichneten Stämme stammen aus der Stammsammlung der BASF AG.

### Beispiel 2

### Herstellung von 3-Hydroxyphenylessigsäure im Schüttelkolben

Ein kleines Mycelstück von Rhizoctonia solani (DSM 852) wurde in 250-ml-Erlenmeyerkolben mit Schikane mit 30 ml des folgenden Mediums angeimpft:

### Medium A:

| | |
|---|---|
| 20 g/l | Glucose |
| 5 g/l | Hefeextrakt (DIFCO) |
| 5 g/l | (NH₄)₂ SO₄ |
| 0,5 g/l | MgSO₄ x 7 H₂O |
| 0,05 g/l | MnSO₄ x 4 H₂O |
| 2 ml/l | Spurenelementlösung |
| 3 g/l | Carbopol 946® (hochmolekulares Carboxyvinylpolymer) |
| 1,5 g/l | KH₂PO₄ |
| 3,6 g/l | K₂HPO₄ |

### Spurenelementlösung:

| | |
|---|---|
| 200 mg/l | Eisen(II)sulfat-1-hydrat |
| 10 mg/l | Zink(II)sulfat-4-hydrat |
| 3 mg/l | Manganchlorid-4-hydrat |
| 30 mg/l | Borsäure |
| 20 mg/l | Cobalt(II)chlorid-6-hydrat |
| 1 mg/l | Kupfer(II)chlorid-2-hydrat |
| 2 mg/l | Nickel(II)chlorid-6-hydrat |
| 3 mg/l | Natriummolybdat-2-hydrat |
| 500 mg/l | Ethylendiamintetraessigsäure (EDTA) |

Mit 5 ml dieser Vorkultur wurde eine Hauptkultur angeimpft. Als Hauptkulturmedium wurden 30 ml Medium A ohne Carbopol verwendet. Zusätzlich wurden 10 g/l Phenylessigsäure zugegeben.

Nach 4 Tagen Schütteln bei 25°C und 180 U/min war die Phenylessigsäure komplett umgesetzt.

Die Biomasse wurde darauf durch 5 minütige Zentrifugation mit 4000 U/min geerntet und in frisches Medium A mit 20 g/l Phenylessigsäure angeimpft. Nach vollständigem Umsatz der Phenylessigäure (nach 7 Tagen) wurde dieser Vorgang wiederholt und die Biomasse in frisches Medium A mit 30 g/l Phenylessigsäure überführt. Nach 10 Tagen war die Phenylessigsäure komplett umgesetzt. Auf diese Weise wurde die Biomasse bis zu 4 mal ohne erkennbaren Aktivitätsverlust wiederverwendet.

Direkt nach Beendigung des Umsatzes enthielten die Kulturen neben 3-Hydroxyphenylessigsäure noch circa 0,5 % 2-Hydroxyphenylessigsäure und bis zu 4 % 4-Hydroxyphenylessigsäure. Der Anteil an 4-Hydroxyphenylessigsäure konnte auf 0 - 1 % vermindert werden, indem die Kultur nach dem Verschwinden der Phenylessigsäure noch einen Tag weitergeführt wurde. Die Konzentration an 2- und 3-Hydroxyphenylessigsäure wurde dadurch nicht beeinflußt.

### Beispiel 3

### Herstellung von 3-Hydroxyphenylessigsäure durch wiederholte 10-1-Fermentationen

### Medium B:

| | |
|---|---|
| 20 g/l | Glucose |
| 7,5 g/l | Hefeextrakt (Fould Springer, 65 %) |
| 5 g/l | (NH₄)₂SO₄ |
| 0,5 g/l | MgSO₄ x 7 H₂O |
| 0,05 g/l | MnSO₄ x H₂O |
| 2 ml/l | Spurenelementlösung |
| 1 g/l | Antischaum P 2000 |
| 1,5 g/l | KH₂PO₄ |
| 3,6 g/l | K₂HPO₄ |

### Vorkultur:

3 x 330 ml Medium B in Rundkolben mit Schikane wurden mit je 1/3 einer mit DSM 852 bewachsenen Agarplatte, zerkleinert mit einem Ultraturrax, angeimpft und 3 Tage bei 25°C mit 180 U/min geschüttelt.

### 10-1-Fermentation:

Mit der Vorkultur wurde ein 10-1-Fermenter mit Medium B und zusätzlich 10 g/l Phenylessigsäure angeimpft. Der Fermenter wurde mit 100 Upm gerührt und mit 0,5 Volumen Luft pro Volumen Reaktor pro Minute (vvm) begast. Während der Fermentation wurde die Drehzahl schrittweise auf 200 Upm und die Begasung auf 1 vvm erhöht.

Nach 88 Stunden war die gesamte Phenylessigsäure umgesetzt. 9 l des Fermenterinhalts wurden abgelassen und durch frisches Medium B ersetzt. Die Phenylessigsäurekonzentration wurde durch Zugabe einer 25 gew.-%igen Phenylessigsäurelösung (in Wasser, mit NaOH auf pH 7 gestellt) auf 10 g/l eingestellt.

Während der Fermentation wurden 2 mal je 50 g Phenylessigsäure zudosiert. Bei Bedarf wurde Glucose nachgefüttert. Nach 165 Stunden war die gesamte Phenylessigsäure umgesetzt. Die Endkonzentration an 3-Hydroxyphenylessigsäure betrug 26 g/l.

Wie oben beschrieben wurde der Fermenter bis auf einen Rest von 1 l abgelassen und mit frischem Medium B aufgefüllt. Während 165 Stunden Fermentation wurden 4 x 50 g Phenylessigsäure zudosiert. Die Endkonzentration an 3-Hydroxyphenylessigsäure betrug 30,6 g/l.

Der Fermenter wurde wiederum bis auf 1 l abgepumpt.

Isolierung von 3-Hydroxyphenylessigsäure aus der Fermentationsbrühe der 3. Fermentation:

Neun Liter Fermentationsbrühe wurden abzentrifugiert (5000 U/min, 20 min), der Niederschlag mit 1 l Wasser gewaschen und nochmals abzentrifugiert. Der Überstand der beiden Zentrifugationen (ca. 7 1) wurde mit H₂SO₄ auf pH 2 eingestellt und 2 mal mit dem gleichen Volumen tert.-Butylmethylether extrahiert. Die organische Phase wurde zur Trockene eingedampft und ergab einen Rückstand von 250 g, der zu 94 % aus 3-Hydroxyphenylessigsäure bestand.

## Patentansprüche

1. Verfahren zur Herstellung von 3-Hydroxyphenylessigsäure, dadurch gekennzeichnet, daß man einen Pilz, ausgewählt aus der Gruppe der Gattungen Rhizoctonia, Ceratobasidium und Pellicularia in Gegenwart eines üblichen Nährmediums, dem eine Phenylverbindung der allgemeinen Formel I, 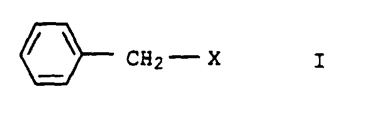 in der der Substituent folgende Bedeutung hat:
X = COOR, CH₂OH, CH₂NH₂, 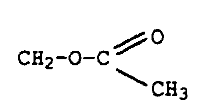
R = Methyl, Ethyl, Wasserstoff, Natrium, Kalium, Ammonium,
zugesetzt wird, züchtet.

## Claims

1. A process for preparing 3-hydroxyphenylacetic acid, which comprises cultivating a fungus selected from the group of genera Rhizoctonia, Ceratobasidium and Pellicularia in the presence of a conventional nutrient medium to which is added a phenyl compound of the formula I 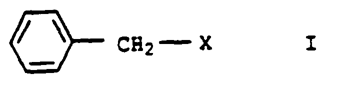 where
X is COOR, CH₂OH, CH₂NH₂ or 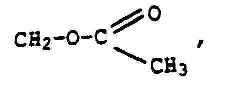
R is methyl, ethyl, hydrogen, sodium, potassium or ammonium.

## Revendications

1. Procédé de préparation d'acide 3-hydroxyphényl-acétique, caractérisé par le fait que l'on sélectionne un champignon, choisi dans le groupe des espèces Rhizoctonia, Ceratobasidium et Pellicularia, en présence d'un milieu de culture usuel auquel on a ajouté un composé phénylique de formule générale I 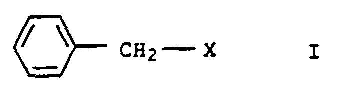 dans laquelle les substituants ont la signification suivante
X = COOR, CH₂OH, CH₂NH₂, 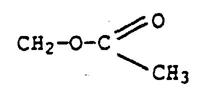
R = méthyle, éthyle, hydrogène, sodium, potassium, ammonium.
